# EUROPÄISCHE PATENTANMELDUNG

(11) **EP 4 813 441 A1**
(43) Veröffentlichungstag der Anmeldung: **30.09.2026**
(21) Anmeldenummer: 25167196.2
(22) Anmeldetag: 28.03.2025
(51) Int. Cl.: A61N 1/04, A61N 1/36

(54) **SYSTEM ZUR STIMULATION VON KÖRPERREGIONEN EINES NUTZERS**

(71) Anmelder: Beurer GmbH, 89077 Ulm (DE)
(72) Erfinder: WEBER, Sandra, 89293 Kellmünz an der Iller (DE); RAITH, Lara, 72589 Westerheim (DE)
(74) Vertreter: Maiwald GmbH

(57) **Zusammenfassung**

Die Erfindung betrifft ein System (10) zur Stimulation, insbesondere zur wahlweise transkutanen elektrischen Nervenstimulation, TENS, elektrischen Muskelstimulation, EMS, und/oder Massagestimulation, von Körperregionen eines Nutzers, wobei das System (10) aufweist: zumindest eine Elektrodeneinheit (14a), welche zur Anordnung an der Haut des Nutzers und zur Übertragung von elektrischen Impulsen an die Haut eingerichtet ist, eine Bedieneinheit (12), welche zum Erfassen zumindest einer Bedieneingabe eines Nutzers zur Auswahl einer zu stimulierenden Körperregion (13b) aus einer Mehrzahl von zur Stimulation auswählbaren Körperregionen eingerichtet ist, und eine Steuereinheit (15), welche zur Steuerung der elektrischen Impulse gemäß der zumindest einen Bedieneingabe derart eingerichtet ist, dass abhängig von der ausgewählten zu stimulierenden Körperregion (13b) automatisch ein Steuerprogramm zur Steuerung der elektrischen Impulse ausgewählt wird.

## Beschreibung

### TECHNISCHES GEBIET

Die Erfindung betrifft allgemein das Gebiet der Stimulation von Körperregionen eines Nutzers, insbesondere durch transkutane elektrische Nervenstimulation, TENS, elektrische Muskelstimulation, EMS, und/oder Massagestimulation. Insbesondere betrifft die Erfindung ein System zur Stimulation von Körperregionen eines Nutzers, ein Verfahren zur Steuerung eines Systems zur Stimulation einer Körperregion eines Nutzers, ein Computerprogramm sowie ein computerlesbares Speichermedium.

### HINTERGRUND

Für die Stimulation von Körperregionen eines Nutzers durch verschiedene Techniken oder Stimulationsprogramme, wie beispielsweise TENS, EMS, oder Massage, sind diverse Systeme oder Geräte bekannt. Gerade im Heimgebrauch finden oft Kombigeräte Anwendung, die mehrere Techniken oder Stimulationsprogramme miteinander kombinieren.

Bei bekannten Systeme oder Geräten ist die Bedienung des Geräts mit der Auswahl von entsprechenden Steuerprogrammen für verschiedene Körperregionen, die der Nutzer durch das Gerät mittels elektrischen Impulsen stimulieren möchte, relativ kompliziert und ohne das mehrfache Lesen eines entsprechenden Handbuchs oder ohne eine entsprechende Einweisung teils nur schwer und ggf. nicht optimal umsetzbar. Gerade das Anwenden eines passenden Steuerprogramms für den vorliegenden Anwendungsfall einer aktuellen Beschwerde des Nutzers oder allgemein für eine nutzerseitig gewünschte Stimulation ist ohne entsprechendes Vorwissen nur schwer möglich.

Hinzu kommt oftmals eine unübersichtliche oder komplizierte Bedienung bei entsprechenden Systemen oder Geräten. Oft kommt es dabei beispielsweise zur Doppelbelegung von Tasten mit entsprechenden Funktionen oder zur Verwendung von sehr vielen verschiedenen Tasten an der Bedieneinheit.

### ZUSAMMENFASSUNG

Entsprechend wäre es wünschenswert, ein System oder Gerät zur Stimulation von Körperregionen eines Nutzers bereitzustellen, welches die vorstehenden Nachteile zumindest teilweise behebt.

Mit der vorliegenden Erfindung können in vorteilhafter Art und Weise ein System zur Stimulation von Körperregionen eines Nutzers, ein Verfahren zur Steuerung eines solchen Systems zur Stimulation von Körperregionen eines Nutzers, ein Computerprogramm sowie ein computerlesbares Speichermedium bereitgestellt werden, die eine intuitive Bedienung des Systems und eine für den jeweiligen Nutzer optimierte Stimulation ohne nutzerseitiges Vorwissen ermöglichen.

Die Erfindung ist in den unabhängigen Patentansprüchen definiert. Vorteilhafte Weiterbildungen und/oder Ausführungsformen sind in den abhängigen Patentansprüchen sowie der vorstehenden und nachstehenden Beschreibung angegeben.

Ein erster Aspekt der Erfindung betrifft ein System zur Stimulation, insbesondere zur wahlweise transkutanen elektrischen Nervenstimulation, TENS, elektrischen Muskelstimulation, EMS, und/oder Massagestimulation, von Körperregionen eines Nutzers. Das System weist zumindest eine Elektrodeneinheit auf, welche zur Anordnung an der Haut des Nutzers und zur Übertragung von elektrischen Impulsen an die Haut eingerichtet ist. Ferner weist das System eine Bedieneinheit auf, welche zum Erfassen zumindest einer Bedieneingabe eines Nutzers zur Auswahl einer zu stimulierenden Körperregion aus einer Mehrzahl von zur Stimulation auswählbaren Körperregionen eingerichtet ist. Zudem weist das System eine Steuereinheit auf, welche zur Steuerung der elektrischen Impulse gemäß der zumindest einen Bedieneingabe derart eingerichtet ist, dass abhängig von der ausgewählten zu stimulierenden Körperregion automatisch ein Steuerprogramm zur Steuerung der elektrischen Impulse ausgewählt wird.

Dementsprechend wird erfindungsgemäß ein System mit einer Steuereinheit bereitgestellt, die durch eine automatische Auswahl eines Steuerprogramms, basierend auf einer von dem Nutzer zur Stimulation ausgewählten Körperregion, eine Steuerung der elektrischen Impulse zur optimierten Stimulation des Nutzers ermöglicht. Insbesondere kann die automatische Auswahl durch die Steuereinheit zumindest die vom Nutzer ausgewählte Körperregion berücksichtigen bzw. darauf abgestimmt sein, sodass ein Steuerprogramm automatisch gewählt und das System demgemäß betrieben wird, welches eine verbesserte Stimulation im vorliegenden Anwendungsfall des Nutzers ermöglicht. Zudem wird so eine intuitive Bedienung ermöglicht, weil der Nutzer nicht noch zwischen einer Vielzahl von Steuerprogrammen wählen muss, was ohne Vorwissen schwierig ist. Damit wird insbesondere ein Stimulationssystem mit nutzergeführter Auswahl von Körperregionen und automatisierter Steuerung der Stimulationsparameter bereitgestellt.

Das System kann durch ein oder mehrere Geräte gebildet sein. Insbesondere kann das System zumindest ein Stimulationsgerät umfassen, wobei wenigstens die Steuereinheit und optional die Bedieneinheit in dem Stimulationsgerät umfasst sind. Weiters kann die zumindest eine Elektrodeneinheit damit gekoppelt oder verbunden sein. Insbesondere kann die zumindest eine Elektrodeneinheit damit verbindbar sein. So ist es beispielsweise möglich, dass das System aus mehreren Geräten oder Einheiten zusammenbaubar oder zusammensetzbar ist, beispielsweise durch Verbinden der zumindest einen Elektrodeneinheit mit dem Stimulationsgerät, insbesondere drahtlos oder drahtgebunden. Beispielsweise kann das System als ein Kombisystem oder Kombigerät ausgebildet sein, welches wahlweise für TENS, EMS, und/oder Massagestimulation ausgebildet ist oder zwei beliebige der drei vorgenannten Stimulationsarten. Die Auswahl der jeweiligen Stimulationskategorie TENS, EMS und/oder Massagestimulation kann nutzerseitig über die Bedieneinheit erfolgen.

EMS wird typischerweise verwendet, um Muskelgewebe zu stimulieren, zu dehnen und zu kräftigen. EMS verwendet elektrische Impulse, die das Muskelgewebe dazu anregen, sich zusammenzuziehen. Dies verbessert die Durchblutung und hilft bei der Stärkung und Rehabilitation geschwächter Muskeln. TENS zielt darauf ab, Schmerzen zu lindern, indem es elektrische Impulse an die Nervenenden in der Haut sendet. Dabei wird das Nervensystem stimuliert und die Schmerzen werden so blockiert, dass sie nicht zum Gehirn durchdringen. Massagestimulation verwendet allgemein elektrische Impulse zur stärkeren Stimulierung von Muskulatur, die einen Massageeffekt bei der betroffenen Körperregion hervorrufen sollen, die mit einem in Empfindung und Wirkung an eine reale Massage angelehnten Programm Muskelverspannungen abbaut.

Das System kann insbesondere mehrere Elektrodeneinheiten umfassen, die jeweils an die Haut des Nutzers angeordnet werden können, um elektrische Impulse auf verschiedene Körperpartien und/oder großflächig auf dem Körper des Nutzers zu übertragen.

Die Bedieneinheit kann zumindest ein Bedienfeld oder zumindest eine Bedientaste umfassen, welche die Bedieneingabe des Nutzers erfassen kann. Möglich sind verschiedenartige Ausführungen wie ein oder mehrere Berührbildschirme, berührempfindliche Tasten, druckempfindliche Tasten, akustische Bedieneinheiten zum Erhalten von akustischen Bedieneingaben, und dergleichen.

Das System kann zudem eine Anzeige umfassen. Die Anzeige kann als Teil der Bedieneinheit oder zusätzlich zu der Bedieneinheit von dem System umfasst sein. Die Anzeige oder der Bildschirm kann eine Menüführung aufweisen oder darstellen, welche die Bedienung durch den Nutzer unterstützt. Beispielsweise kann der Bildschirm zu der zumindest einen Bedieneingabe oder jeder Bedieneingabe, die vom Nutzer erwartet wird, eine Bedienaufforderungen anzeigen. Beispielsweise kann die Anzeige dazu eingerichtet sein, die erfasste zumindest eine Bedieneingabe anzuzeigen oder zu bestätigen, z.B. indem zur nächsten Bedienaufforderungen gewechselt wird oder allgemein ein Statusbildschirm oder dergleichen nach erfolgter Bedieneingabe angezeigt wird, indem relevante Parameter bezüglich der Stimulation angezeigt werden. Insbesondere kann die Anzeige im Rahmen der Menüführung die jeweilige erwartete Bedieneingabe oder Bedienaufforderungen anzeigen, also etwa, dass eine Körperregion zur Stimulation durch den Nutzer ausgewählt werden muss. Dabei kann die Anzeige die verschiedenen zur Stimulation auswählbaren Körperregionen im Rahmen einer graphischen Darstellung anzeigen. Die graphische Darstellung kann beispielsweise die eines zumindest teilweisen menschlichen Körpers umfassen. Mittels der Bedieneinheit kann es ermöglicht sein, verschiedene Körperregionen zur Stimulation auszuwählen, wobei für eine aktuell ausgewählte Körperregion die betroffene Körperregion in der graphischen Darstellung aufleuchten oder anderweitig optisch angezeigt werden kann. Mittels der Bedieneinheit kann es zudem ermöglicht sein, die optisch angezeigte Körperregion zu bestätigen, wodurch die zumindest eine Bedieneinheit zur Auswahl einer zu stimulierenden Körperregion aus der Mehrzahl von zur Stimulation auswählbaren Körperregionen von der Bedieneinheit erhalten bzw. erfasst werden kann.

Die Steuereinheit ist dazu eingerichtet, die elektrischen Impulse gemäß dem automatisch ausgewählten Steuerprogramm zu steuern. Dabei erfolgt die Steuerung gemäß einem Steuerprogramm, welches ein oder mehrere Einstellungen umfassen kann, gemäß derer die elektrischen Impulse gesteuert werden. Dabei wählt die Steuereinheit automatisch abhängig, insbesondere anhand, der vom Nutzer ausgewählten zu stimulierenden Körperregion dasjenige Steuerprogramm aus insbesondere einer Vielzahl von Steuerprogrammen für unterschiedliche zu stimulierende Körperregionen aus, welches angewendet werden soll und entsprechend auch angewendet wird. Automatisch meint dabei, dass die Steuereinheit das Steuerprogramm ohne zusätzliche oder weitere Nutzeraktion auswählt, welche die Steuerung seitens des Nutzers nur verkomplizieren würde. Die Auswahl umfasst dabei insbesondere auch die Bestätigung bzw. Anwendung des entsprechenden Steuerprogramms, insbesondere ohne vorherige Nutzerbedienung oder nutzerseitige Abfrage. Vielmehr trifft die Steuereinheit nach Bedieneingabe des Nutzers bzgl. der zu stimulierenden Körperregion automatisch das entsprechende Steuerprogramm und wendet es auch insbesondere an. Insbesondere ist die Steuereinheit entsprechend dazu eingerichtet, auf Basis des automatisch ausgewählten Steuerprogramms die elektrischen Impulse zu steuern bzw. das Steuerprogramm auszuführen.

Ganz besonders kann im Rahmen der Bedieneingabe bzw. des Auswahlschritts der zu stimulierenden Körperregion jeder Körperregion genau ein einziges und/oder vordefiniertes Steuerprogramm hinterlegt oder zugeordnet sein, welches entsprechend automatisch ausgewählt wird. Dies bedingt jedoch nicht zwangsläufig, wenn auch optional möglich, dass im System für jede auswählbare Körperregion nur ein Steuerprogramm hinterlegt ist. Vielmehr ist es möglich, weitere Auswahlschritte im Rahmen der Bedienung zu haben, welche die automatische Auswahl des Steuerprogramms bedingen können. So können insbesondere vorher weitere Bedieneingaben erfasst werden, um andere Einstellungen zu treffen, insbesondere die nachstehend erwähnte Auswahl der Stimulationskategorie und/oder Unterstimulationskategorie. Abhängig von dieser vorherigen Auswahl durch nutzerseitige Bedieneingabe kann bei dem Auswahlschritt der zu stimulierenden Körperregion zwar weiterhin jeder Körperregion genau ein einziges und/oder vordefiniertes Steuerprogramm hinterlegt oder zugeordnet sein, jedoch kann sich dieses Steuerprogramm für dieselbe Körperregion je nach zuvor ausgewählter Stimulationskategorie und/oder Unterstimulationskategorie unterscheiden. Entsprechend kann die vorherige Auswahl bzw. Bedieneingabe zur Wahl der Stimulationskategorie und/oder zur Wahl der Unterstimulationskategorie die Vielzahl von Steuerprogrammen für unterschiedliche zu stimulierende Körperregionen, aus denen die Steuereinheit abhängig von der gewählten zu stimulierenden Körperregion automatisch auswählt, bedingen.

Beispielsweise kann die Bedieneinheit ferner zum Erfassen zumindest einer Bedieneingabe eines Nutzers zur Auswahl einer Stimulationskategorie aus einer Mehrzahl von Stimulationskategorien eingerichtet sein. Dabei kann das Steuerprogramm ferner abhängig von der ausgewählten Stimulationskategorie automatisch ausgewählt werden.

Beispielsweise kann die Mehrzahl von Stimulationskategorien zumindest zwei der folgenden aufweisen: TENS, EMS, und/oder Massagestimulation. Insbesondere können zwei beliebige der vorstehenden Stimulationskategorien umfasst sein oder aber alle drei. Entsprechend kann der Nutzer durch eine weitere, insbesondere vorherige Bedieneingabe auf oder an der Bedieneinheit eine der jeweiligen Stimulationskategorien auswählen, um entsprechend eine Auswahl der verschiedene Funktionalitäten eines derartigen Kombisystems oder Kombigeräts bereitzustellen. Das automatisch ausgewählte Steuerprogramm bei der Erfassung der vom Nutzer ausgewählten Körperregion kann entsprechend den unterschiedlichen Stimulationskategorien unterschiedlich ausfallen, wird jedoch nutzerseitig durch eine vorherige Auswahl der Stimulationskategorie vorgegeben und nicht aktiv oder im Schritt der Auswahl der zu stimulierenden Körperregion vom Nutzer gewählt, sondern erfolgt wie beschrieben automatisch durch die Steuereinheit.

Beispielsweise kann die Bedieneinheit ferner zum Erfassen zumindest einer Bedieneingabe eines Nutzers zur Auswahl einer Unterstimulationskategorie aus zwei Unterstimulationskategorien eingerichtet sein. Dabei kann das Steuerprogramm ferner abhängig von der ausgewählten Unterstimulationskategorie ausgewählt werden. Dabei können die zumindest zwei Unterstimulationskategorien eine chronische Unterstimulationskategorie und eine akute Unterstimulationskategorie umfassen. Insbesondere kann die Auswahl der Unterstimulationskategorie erfolgen bzw. das Erfassen der entsprechenden Bedieneingabe erfolgen, falls TENS als Stimulationskategorie gewählt worden ist. Zusätzlich oder alternativ ist aber auch möglich, dass Unterstimulationskategorie ausgewählt werden können abhängig von den anderen Stimulationskategorien. So kann beispielsweise vorgesehen sein, dass bei Auswahl von EMS als Stimulationskategorie zwischen Unterstimulationskategorien zum Muskelaufbau und zur Regeneration ausgewählt werden kann. Bei Auswahl von Massage als Stimulationsprogramm kann beispielsweise als Unterstimulationskategorie Klopfmassage und klassische Massage auswählbar sein. Dann kann eine entsprechende Bedienaufforderung auf der Anzeige erfolgen, welche auch eine Bedienung zur Auswahl der Unterstimulationskategorie ermöglicht. Die chronische Unterstimulationskategorie kann beispielsweise für chronische Beschwerden oder Schmerzen eingerichtet sein. Insbesondere kann dem Nutzer auf der Anzeige ein entsprechendes Symbol und/oder das Wort chronisch oder ähnlich angezeigt werden, damit der Nutzer die chronische Unterstimulationskategorie auswählen kann. Die akute Unterstimulationskategorie kann beispielsweise für akute Beschwerden oder Schmerzen geeignet sein. Insbesondere kann dem Nutzer auf der Anzeige ein entsprechendes Symbol und/oder das Wort akut oder ähnlich angezeigt werden, damit der Nutzer die akute Unterstimulationskategorie auswählen kann. Entsprechend kann der Nutzer durch eine weitere Bedieneingabe, insbesondere nach Auswahl der Stimulationskategorie und vor Auswahl der zu stimulierenden Körperregion, eine etwas feinere Einstellung der Stimulation zur Behandlung chronischer oder akuter Beschwerden vornehmen. Das automatisch ausgewählte Steuerprogramm bei der Erfassung der vom Nutzer ausgewählten Körperregion kann entsprechend den unterschiedlichen Unterstimulationskategorien unterschiedlich ausfallen, wird jedoch nutzerseitig durch eine vorherige Auswahl der Unterstimulationskategorie vorgegeben und nicht aktiv oder im Schritt der Auswahl der zu stimulierenden Körperregion vom Nutzer gewählt, sondern erfolgt wie beschrieben automatisch durch die Steuereinheit.

Beispielsweise kann das automatisch ausgewählte Steuerprogramm eine Frequenz, eine Pulsweite, einer Modulation, eine Einschaltzeit und/oder eine Ausschaltzeit der elektrischen Impulse vorgeben. Auch die Vorgabe mehrerer Frequenzen, Pulsweiten, Modulationen usw. ist möglich. Die Frequenz gibt die Anzahl der Einzelimpulse pro Zeiteinheit, insbesondere pro Sekunde in Hz an. Die Pulsweite bezeichnet grundsätzlich die Dauer des Impulses, insbesondere in Mikrosekunden. Die Fläche unter dem Impuls ist die Energie, die durch den Impuls in die Körperregion übertragen wird. Die Modulation bezeichnet die Veränderung von Pulsweite und/oder Frequenz über die Zeit. Die Einschaltzeit und Ausschaltzeit können beispielsweise die gesamte Stimulationsdauer des Steuerprogramms bestimmen. Durch Vorgabe eines oder mehrerer der vorgenannten Parameter in dem automatisch ausgewählten Steuerprogramm kann entsprechend eine gezielte Stimulation für eine bestimmte Körperregion und optional je nach Auswahl der Stimulationskategorie und/oder Unterstimulationskategorie erzielt werden. Die bei der automatischen Auswahl zur Verfügung stehenden unterschiedlichen Steuerprogramme, wobei jedes einer Körperregion zugeordnet ist, können sich in zumindest einem oder mehreren der vorgenannten Parameter unterscheiden, sodass jedes Steuerprogramm gezielt für eine bestimmte Körperregion und ggf. eine Stimulationskategorie und/oder Unterstimulationskategorie vordefiniert ist.

Es kann jedoch auch möglich sein, dass der Nutzer nach Auswahl des Steuerprogramms zumindest einen oder mehrere Stimulationsparameter auch einstellen kann. Typischerweise können diese Stimulationsparameter jedoch nicht derart fein oder detailliert sein, wie sie in dem Steuerprogramm vordefiniert sind. Stattdessen kann beispielsweise vorgesehen sein, dass die Bedieneinheit eine Bedieneingabe bezüglich einer Wahl einer Intensität vom Nutzer erfassen kann. Die Intensität als Stimulationsparameter kann beispielsweise in vordefinierten Intensitätsstufen anpassbar oder wählbar sein. Die Intensität kann sich beispielsweise aus mehreren der vorgenannten Parameter in dem Steuerprogramm oder anderen Parametern zusammensetzen. Dadurch kann es dem Nutzer ermöglicht werden, trotz automatisch ausgewähltem Steuerprogramm Einstellungen vorzunehmen, die die Steuerung der elektrischen Impulse auf Basis des automatisch ausgewählten Steuerprogramms beeinflussen bzw. verändern. Andererseits kann aber vorgesehen sein, dass das Steuerprogramm an sich nicht vom Nutzer bzw. nicht durch Bedieneingabe veränderbar ist, also nicht gewechselt werden kann. Dadurch wird eine unnötige Komplexität der Steuerung durch den Nutzer vermieden.

Beispielsweise können die zur Stimulation auswählbaren Körperregionen in der Steuereinheit vorgegebene Körperregionen sein, welche zusammenhängende Körperpartien und/oder voneinander getrennte Körperpartien des Nutzers umfassen. Entsprechend wird die nutzerseitige Bedienung auf vorgegebene und dadurch leicht auswählbare Körperregionen beschränkt. Dabei können die Körperpartien zusammenhängen bzw. aneinander liegen, so beispielsweise Rücken und Schulter, oder voneinander getrennt sein, beispielsweise Arme. Auf einer entsprechenden Anzeige können die zur Auswahl stehenden Körperregionen jeweils in einer graphischen Darstellung des menschlichen Körpers sichtbar gemacht werden. Insbesondere kann durch eine entsprechende Bedienung zwischen den verschiedenen zur Auswahl stehenden Körperregionen gewechselt werden und die gewünschte Körperregion kann dann bestätigt werden.

Beispielsweise kann die Bedieneinheit von einem Endgerät des Nutzers, einem Handgerät oder einem Tischgerät umfasst sein. So kann beispielsweise vorgesehen sein, dass ein Endgerät des Nutzers, wie ein Smartphone oder Tablet-Computer, mit der Steuereinheit und/oder der zumindest einen Elektrodeneinheit oder grundsätzlich mit dem System drahtlos oder drahtgebunden verbindbar ist. Auf dem Endgerät kann dabei eine entsprechende Bediensoftware, beispielsweise in Form einer App, installiert sein, um die Bedieneingaben zu erfassen und an die Steuereinheit weiterzuleiten. Alternativ kann es sich bei der Bedieneinheit um ein Handgerät handeln bzw. kann sich die Bedieneinheit an einem Handgerät befinden, also es sich um ein in der Hand des Nutzers haltbares Gerät handeln. Das Handgerät kann dabei das Stimulationsgerät sein. Auch besteht die Möglichkeit der Ausbildung der Bedieneinheit in einem Tischgerät, welches als auf einem Tisch abstellbar ist, von dem aus es sich bedienen lässt. Auch das Tischgerät kann entsprechend als Stimulationsgerät ausgebildet sein, also ferner mit der Steuereinheit und beispielsweise einer Energieversorgungseinheit zum Bereitstellen des Stroms zur Erzeugung der elektrischen Energie. Ferner kann das Stimulationsgerät grundsätzlich mehrere Kanäle zum Anschluss der Elektrodeneinheiten daran aufweisen. Die Kanäle können mittels der Bedieneinheit in der Intensität der elektrischen Impulse einzeln steuerbar sein.

Beispielsweise kann die Bedieneinheit mit der zumindest einen Elektrodeneinheit verbindbar oder verbunden sein. Zudem kann die Bedieneinheit dazu eingerichtet sein, die elektrischen Impulse zu erzeugen und über die Verbindung mit der zumindest einen Elektrodeneinheit an die Haut zu übertragen. Eine Elektrodeneinheit kann insbesondere zumindest oder exakt zwei Elektroden aufweisen, die an denselben Kanal angeschlossen sein können. Dadurch ist eine Stimulation ermöglicht, bei der die elektrischen Impulse als Strom zwischen den zwei Anordnungspunkten auf der Haut des Nutzers strömen. Insbesondere kann eine der Elektroden den elektrischen Impuls bzw. damit einhergehenden Strom an die Haut bzw. zum Nutzer übertragen und die andere den elektrischen Impuls bzw. Strom an das System zurückführen. Dabei sind drahtgebundene oder drahtlose Verbindung zwischen einer oder mehreren Elektrodeneinheiten und der Bedieneinheit möglich. So kann beispielsweise eine drahtlose Verbindungstechnologie benutzt werden, um entsprechende Steuersignale gemäß dem Steuerprogramm an die eine oder mehreren Elektrodeneinheiten zu senden, wo diese in entsprechende elektrische Impulse von entsprechenden Aktuatoren, wie beispielsweise Batterien, umgesetzt werden. Alternativ ist selbstverständlich möglich, dass die Bedieneinheit selbst bzw. eine Energiequelle wie Batterie oder Energie aus einem Stromanschluss die Erzeugung der elektrischen Impulse ermöglicht, welche drahtgebunden an die Elektrodeneinheiten weitergeleitet wird. Beispielsweise kann das System insbesondere als ein kompaktes Handgerät mit der Bedieneinheit, der Steuereinheit, der Verbindung zu den Elektrodeneinheiten und optional der Anzeige ausgeführt sein.

Beispielsweise kann die Bedieneinheit eine Anzeige aufweisen, auf welcher die zur Stimulation auswählbaren Körperregionen in Abhängigkeit von der zumindest einen Bedieneingabe des Nutzers an der Bedieneinheit angezeigt werden. Entsprechend können die Körperregionen zur intuitiveren Auswahl visualisiert werden, damit der Nutzer die richtige Auswahl derjenigen Körperregion treffen kann, bei der er beispielsweise Beschwerden hat oder die er grundsätzlich stimulieren möchte.

Beispielsweise kann die Bedieneinheit eine Mehrzahl von Tastenfeldern zur Erfassung der zumindest einen Bedieneingabe des Nutzers aufweisen. Alternativ sind aber auch andere Bedieneinheiten, z.B. mit Berührbildschirmen möglich. Eine begrenzte Anzahl von Tastenfeldern ermöglicht jedoch, insbesondere in der nachstehenden Weise, eine besonders intuitive und erleichterte Nutzerbedienung und dadurch eine letztendlich optimiert eingestellte Stimulation der gewünschten Körperregion des Nutzers.

Dabei kann die Bedieneinheit zwei erste Auswahl-Tastenfelder zur Auswahl der zu stimulierenden Körperregion und ein Bestätigungs-Tastenfeld zur Bestätigung der ausgewählten Körperregion und/oder zur Einstellung von einem Stimulationsparameter in dem automatisch ausgewählten Steuerprogramm aufweisen. Die zwei ersten Auswahl-Tastenfelder können beispielsweise mit < und > oder ähnlichen bezeichnet sein. Das Bestätigungs-Tastenfeld kann entsprechend ebenfalls mit einer entsprechenden Bestätigungsindikation einem OK-Zeichen, einem I/O-Zeichen oder Ein/Aus-Zeichen oder ähnlich beschriftet sein. Das Bestätigungs-Tastenfeld kann auch zum Ein- und Ausschalten des Systems eingerichtet sein, beispielsweise durch langes Drücken bzw. Halten. Dadurch ist es dem Nutzer, insbesondere in Verbindung mit der Anzeige, möglich, eine besonders einfache Auswahl der zu stimulierenden Körperregion vorzunehmen. Bei dem Stimulationsparameter kann es sich insbesondere um die Intensität handeln. Dadurch wird die Benutzerführung anhand der zwei ersten Auswahl-Tastenfelder auf eine besonders intuitive und einfache Möglichkeit der Intensitätseinstellung beschränkt. Auch kann zusätzlich ein Zurück-Tastenfeld vorgesehen sein, um bei der Auswahl zurückzuspringen zu können bzw. eine vorgenommene Auswahl neu vorzunehmen. Auch kann zusätzlich oder gemeinsam mit dem Zurück-Tastenfeld ein Tastenfeld zum Speichern eines Favoritenprogramms vorgesehen sein. Beispielsweise kann es sich um dasselbe Tastenfeld wie das Zurück-Tastenfeld handeln, welches jedoch anders betätigt werden muss, um zwischen der Aktion Zurück und Speichern des Favoritenprogramms zu unterscheiden, beispielsweise durch langes Halten bzw. Drücken des Tastenfelds zum Speichern des Favoritenprogramms. Das Zurück-Tastenfeld kann mit einem entsprechenden Zurück-Zeichen und/oder Favoriten-Zeichen, beispielsweise in der Form eines Herzens, oder ähnlich beschriftet sein.

Ferner kann die Bedieneinheit zwei zweite Auswahl-Tastenfelder aufweisen. Diese können für weitere Auswahlmöglichkeiten und/oder andere Auswahlen bei der Bedienung des Systems genutzt werden können, so beispielsweise zur Einstellung der Intensität der elektrischen Impulse, insbesondere für einen mittels der ersten Auswahl-Tastenfelder auszuwählenden Kanal einer Elektrodeneinheit, sodass hier unterschiedliche Auswahl-Tastenfelder vorteilhaft sind. Die zweiten Auswahl-Tastenfelder können beispielsweise mit Indikationen wie z.B. + und - beschriftet sein, die intuitiv für ein Erhöhen und Senken der Intensität sind.

Grundsätzlich können die jeweiligen Tastenfelder beliebig zueinander angeordnet sein. Beispielsweise kann vorgesehen sein, dass die ersten Auswahl-Tastenfelder und die zweiten Auswahl-Tastenfelder entlang eines gemeinsamen Umfangs, z.B. eines Kreises, eines Quadrats, oder ähnlichem angeordnet sind. Dabei können die ersten Auswahl-Tastenfelder einander gegenüberliegen. Zudem können die zweiten Auswahl-Tastenfelder einander gegenüberliegen. Das Bestätigungs-Tastenfeld kann z.B. mittig der Auswahl-Tastenfelder angeordnet sein. Ein Zurück-Tastenfeld kann oberhalb der Auswahl-Tastenfelder angeordnet sein. Alternativ ist aber auch möglich, die ersten Auswahl-Tastenfelder in einer Reihe nebeneinander anzuordnen und die zweiten Auswahl-Tastenfelder in einer Reihe nebeneinander anzuordnen, wobei eine Reihe über der anderen angeordnet sein kann und das Bestätigungs-Tastenfeld mittig der Auswahl-Tastenfelder angeordnet sein kann. Dadurch wird eine besonders intuitiv und einfach handhabbare Bedienoberfläche bereitgestellt.

Ein zweiter Aspekt der vorliegenden Erfindung betrifft ein Verfahren zur Steuerung des Systems gemäß dem ersten Aspekt der vorliegenden Erfindung zur Stimulation einer Körperregion eines Nutzers, wobei das Verfahren aufweist: Erfassen, an der Bedieneinheit, zumindest einer Bedieneingabe eines Nutzers zur Auswahl einer zu stimulierenden Körperregion aus einer Mehrzahl von zur Stimulation auswählbaren Körperregionen, automatisches Auswählen, durch die Steuereinheit, eines Steuerprogramms zur Steuerung der elektrischen Impulse abhängig von der ausgewählten zu stimulierenden Körperregion, und Steuern der Steuereinheit gemäß dem automatisch ausgewählten Steuerprogramm.

Beispielsweise kann das Verfahren ferner aufweisen: Erfassen, an der Bedieneinheit, zumindest einer Bedieneingabe des Nutzers zur Auswahl einer Stimulationskategorie aus einer Mehrzahl von Stimulationskategorien. Die Mehrzahl von Stimulationskategorien kann beispielsweise zumindest zwei der folgenden aufweisen: TENS, EMS, und/oder Massagestimulation. Ferner kann das Verfahren aufweisen: Erhalten, an der Bedieneinheit, zumindest einer Bedieneingabe des Nutzers zur Auswahl einer Unterstimulationskategorie aus einer Mehrzahl von Unterstimulationskategorien, wobei diese eine chronische Unterstimulationskategorie und eine akute Unterstimulationskategorie umfasst. Das Steuerprogramm kann dabei ferner abhängig von der ausgewählten Stimulationskategorie und/oder Unterstimulationskategorie automatisch ausgewählt werden.

Ein dritter Aspekt der vorliegenden Erfindung betrifft ein Computerprogramm, umfassend Befehle, die bewirken, dass das System das Verfahren nach dem zweiten Aspekt der vorliegenden Erfindung ausführt.

Ein vierter Aspekt der vorliegenden Erfindung betrifft ein computerlesbares Speichermedium, auf dem das Computerprogramm gemäß dem dritten Aspekt der vorliegenden Erfindung gespeichert ist.

Merkmale, Elemente, Funktionen und/oder Vorteile des hierin beschriebenen Feldgeräts können auch auf die hierin beschriebenen Verfahren, das Computerprogrammprodukt und die Trainingsdaten sowie jeweils umgekehrt und in beliebiger Kombination miteinander angewendet werden.

### KURZE BESCHREIBUNG DER FIGUREN

Im Folgenden werden Ausführungsbeispiele der Erfindung unter Bezugnahme auf die beigefügten Figuren beschrieben.

### Figurenliste

- Fig. 1: zeigt eine Draufsicht auf ein System zur Stimulation von Körperregionen eines Nutzers.
- Fig. 2: zeigt eine schematische Ansicht des Systems aus Fig. 1.
- Fig. 3: zeigt schematisch ein Verfahren unter Verwendung des Systems aus Fig. 1 und 2.
- Fig. 4: zeigt eine Draufsicht auf ein gegenüber Fig. 1 alternatives System.
- Fig. 5: zeigt die Anzeige des Systems der Fig. 4 in einem beispielhaften Betrieb.

Ähnliche, ähnlichwirkende, gleiche oder gleichwirkende Elemente in den Figuren können mit ähnlichen oder gleichen Bezugszeichen versehen sein.

### DETAILLIERTE BESCHREIBUNG VON AUSFÜHRUNGSFORMEN

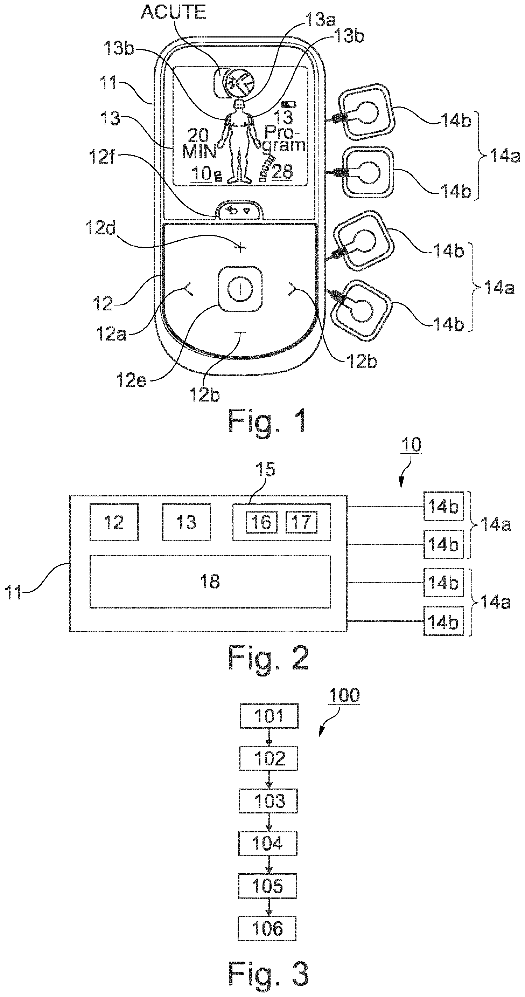

Figur 1 zeigt eine Draufsicht auf ein System 10 zur Stimulation von Körperregionen eines Nutzers. Figur 2 zeigt eine schematische Ansicht des Systems 10.

Wie den Fig. 1 und 2 entnommen werden kann, umfasst das darin gezeigte System 10 beispielhaft ein Handgerät 11 mit einer Bedieneinheit 12. Das Handgerät 11 bildet hier insgesamt ein Stimulationsgerät, insbesondere in Form eines Kombigeräts für verschiedene Stimulationskategorien oder -arten, wie insbesondere TENS, EMS und Massagestimulation. Alternativ kann die Bedieneinheit 12 aber auch ein Endgerät des Nutzers sein oder von einem Tischgerät als Stimulationsgerät umfasst sein.

Das System 10 umfasst vorliegend mehrere Elektrodeneinheiten 14a, welche zur Anordnung an der Haut des Nutzers und zur Übertragung von elektrischen Impulsen an die Haut eingerichtet sind. Rein beispielhaft sind hier zwei Elektrodeneinheiten 14a mit jeweils zwei Elektroden 14b gezeigt, wobei aber auch alternativ mehr oder weniger Elektrodeneinheiten 14a und/oder mehr oder weniger Elektroden 14b zum Einsatz kommen können. Insbesondere können wahlweise durch den Nutzer eine gewünschte oder notwendige Anzahl mit dem Stimulationsgerät bzw. dem Handgerät 11 in diesem Beispiel verbunden werden.

Das System 10 umfasst ferner eine Energieversorgungseinheit 18, welche elektrische Energie zur Verfügung stellen kann, um elektrische Impulse zu erzeugen, welche an die Elektrodeneinheiten 14a gesendet werden können. Beispielsweise kann die Energieversorgungseinheit 18 eine oder mehrere Energiespeicher umfassen, wie z.B. Batterien. Alternativ kann ein Stromanschluss als Energieversorgungseinheit 18 vorgesehen sein, mittels dem das System 10 durch ein Stromkabel an eine elektrische Steckdose angeschlossen werden kann.

Die Erzeugung der elektrischen Impulse wird durch eine Steuereinheit 15 gesteuert, welche hier beispielhaft als ein Computer ausgeführt sein kann oder einen solchen umfassen kann, z.B. in Form eines Mikroprozessors. Die Steuereinheit 15 kann z.B. mit einer Prozessor- oder Verarbeitungseinheit 16 und einem computerlesbaren Speichermedium 17 ausgebildet sein, auf dem ein Computerprogramm gespeichert sein kann, welches Befehle umfasst, die bei Ausführung durch die Prozess- oder Verarbeitungseinheit 15 bzw. den Computer die Ausführung des in Figur 3 schematisch gezeigten Verfahrens 100 bewirken können.

Ferner umfasst das System 10 eine Anzeige 13, welche in der Fig. 1 beispielhaft eine graphische Darstellung eines menschlichen Körpers 13a mit einer von einem Nutzer zur Stimulation ausgewählten Körperregion 13b zeigt. In diesem Beispiel ist die ausgewählte Körperregion 13b durch die voneinander getrennten Körperpartien der Oberarme gekennzeichnet.

Hierneben werden weitere beispielhafte Informationen auf der Anzeige 13 bzw. dem Bildschirm angezeigt, wie beispielsweise ein ausgewähltes und aktuell verwendetes Unterstimulationsprogramm zur akuten Beschwerdebehandlung beim Nutzer im Rahmen einer transkutanen elektrischen Nervenstimulation, bei der der Nutzer die Elektrodeneinheiten 14a an der Haut seiner Oberarme angeordnet hat.

Zudem wird die verbleibende Dauer eines entsprechenden Steuerprogramms, welches hier mit der Nummer 13 bezeichnet ist, angezeigt. Entsprechend dem Steuerprogramm steuert die Steuereinheit 15 die elektrischen Impulse der Elektrodeneinheiten 14a für die akute transkutane elektrische Nervenstimulation in der gezeigten Körperregion 13b.

Außerdem sind in der Anzeige 13 unten links und rechts für beispielhaft zwei unterschiedliche Kanäle, an denen jeweils zwei der beispielhaft zwei Elektrodeneinheiten 14a angeschlossen sind, wie Fig. 1 entnommen werden kann, vom Nutzer gewählte Intensitätsstufen gezeigt. Für einen der Kanäle hat der Nutzer eine Intensität von 10 gewählt und für den anderen der beiden Kanäle eine Intensität von 28 gewählt. Das System 10 kann beispielsweise zur nutzerseitigen Einstellung einer Intensität von 0 bis 50 eingerichtet sein, sodass eine feine Einstellung des Intensitätsgrades der Stimulation ermöglicht ist.

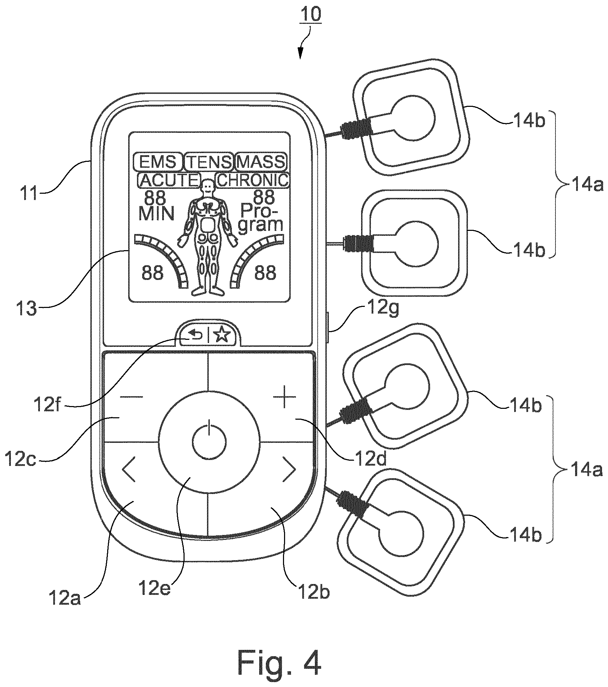

Die Bedieneinheit 12 ist vorliegend besonders übersichtlich und intuitiv bedienbar ausgebildet. Beispielhaft weist die Bedieneinheit 12 dabei sechs Tastenfelder 12a, 12b, 12c, 12d, 12e, 12f auf, mittels welcher Bedieneingaben des Nutzers erfasst werden können. Zusätzlich kann optional noch ein Tastenfeld 12g (siehe Fig. 4) für eine Tastensperre zum Sperren der Tastenfelder 12a, 12b, 12c, 12d, 12e, 12f bzw. der Bedieneinheit 12 vorgesehen sein, welche beispielsweise an einer Seite des Handgeräts 11 angeordnet sein kann. So kann die Bedieneinheit 12 beispielsweise zwei erste Auswahl-Tastenfelder 12a, 12b zur Auswahl der zu stimulierenden Körperregion 13b und ein Bestätigungs-Tastenfeld 12e zur Bestätigung der ausgewählten Körperregion 13b und optional zur Einstellung der Stimulation in dem ausgewählten Steuerprogramm aufweisen. Ferner kann die Bedieneinheit 12 zwei zweite Auswahl-Tastenfelder 12c, 12d aufweisen, die beispielsweise zur Auswahl einer Intensität verwendet werden können, wie nachstehend näher erläutert wird.

Fig. 3 zeigt einen schematischen Ablauf eines Verfahrens 100 zur Stimulation einer Körperregion eines Nutzers mittels des Systems 10, welche eine Bedienabfolge der Bedieneinheit 12 des Nutzers angibt, um das System 10 zur Stimulation einer Körperregion eines Nutzers zu betreiben.

In einem ersten Schritt 101 des Verfahrens 100 trifft der Nutzer bei eingeschaltetem System 10 eine Auswahl einer Stimulationskategorie aus einer Mehrzahl von verfügbaren Stimulationskategorien, insbesondere aus TENS, EMS und Massagestimulation. Dazu kann auf der Anzeige 13 eine entsprechende Bedienaufforderung angezeigt werden.

Bei Bedieneingabe seitens des Nutzers erfasst die Bedieneinheit 12 die Bedieneingabe, beispielsweise auf den ersten Auswahl-Tastenfeldern 12a, 12a zum Selektieren verschiedener Stimulationskategorien und des Bestätigungs-Tastenfelds 12e zur Bestätigung einer selektierten Stimulationskategorie. Der Nutzer kann entsprechend von der Anzeige 13 geführt werden, die die zur Verfügung stehenden Stimulationskategorie anzeigen oder indizieren kann, sodass der Nutzer mit den ersten Tastenfeldern 12a, 12b zwischen den Stimulationskategorien wählen und anschließend mit dem Bestätigungs-Tastenfeld 12e seine Wahl bestätigen kann.

In einem zweiten Schritt 102 des Verfahrens 100 besteht ggf. die Möglichkeit, eine Unterstimulationskategorie aus zwei Unterstimulationskategorien auszuwählen. Dies ist insbesondere dann der Fall, wenn der Nutzer im Schritt 101 TENS gewählt hat. Die beiden Unterstimulationskategorien können z.B. eine chronische und akute Kategorie sein. Die Auswahl kann hier analog dem Schritt 101 mit beispielsweise denselben Tastenfeldern 12a, 12b, 12e erfolgen und anschließend mit dem Bestätigungs-Tastenfeld 12e bestätigt werden.

In einem dritten Schritt 103 des Verfahrens 100 kann der Nutzer anhand der Visualisierung auf der Anzeige 13 mit dem menschlichen Körper 13a eine Körperregion zur Stimulation wählen. Entsprechend kann die Bedieneinheit 12 hier eine Bedieneingabe des Nutzers erfassen, beispielsweise mittels der ersten Auswahl-Tastenfelder 12a, 12b und mit dem Bestätigungs-Tastenfeld 12e, mit welcher eine zu stimulierende Körperregion ausgewählt wird, in dem Beispiel der Fig. 1 die beispielhaft gezeigte Körperregion 13b. Auch hierbei kann der Nutzer anhand der Anzeige 13 geführt werden, z.B. indem verschiedene Körperregionen, die für die Stimulation zur Auswahl stehen, mittels der ersten Auswahl-Tastenfelder 12a, 12b selektiert bzw. zwischen diesen hin und her gewechselt werden kann, wobei die eigentliche Auswahl dann mit dem Bestätigungs-Tastenfeld 12e bestätigt werden kann. Hierneben kann der Nutzer die Elektrodeneinheiten 14a bzw. Elektroden 14b an seiner Haut in der gewählten Körperregion 13b, hier beispielsweise den Oberarmen, befestigen.

In einem vierten Schritt 104 des Verfahrens 100 wählt die Steuereinheit 15 dann automatisch in Abhängigkeit von der während dem Schritt 103 ausgewählten zu stimulierenden Körperregion 13b das Steuerprogramm, welches anschließend zur Steuerung der elektrischen Impulse verwendet wird. Eine nutzerseitige Auswahl oder Bestätigung erfolgt hier nicht mehr. Vielmehr geht die Auswahl, Bestimmung oder Anwendung des jeweiligen Steuerprogramms mit der Auswahl der zu stimulierenden Körperregion automatisch einher. Dadurch wird die Komplexität der Bedienung des Systems 10 reduziert, weil das System 10 ein auf die gewählte Körperregion abgestimmtes Steuerprogramm automatisch anwendet, ohne dem Nutzer hier zusätzliche Auswahlmöglichkeiten oder Optionen zu geben, die zwar eine Individualisierung ermöglichen würden, aber relativ komplex wären, insbesondere bei Nutzern ohne Vorerfahrung oder Vorwissen.

Optional kann in einem fünften Schritt 105 des Verfahrens 100 noch die Intensität durch den Nutzer eingestellt werden, beispielsweise durch Auswahl des einzustellenden Kanals mittels der ersten Tastenfelder 12a, 12b und der Intensitätsstufe bzw. des Intensitätsgrads mittels der zweiten Tastenfelder 12c, 12d. Diese Einstellungen können auch während des Betriebs bzw. der Stimulation vornehmbar sein.

In einem sechsten Schritt 106, oder bereits mit dem Schritt 105 oder davor, erfolgt die Stimulation der ausgewählten Körperregion 13b, an der die Elektrodeneinheiten 14a platziert sind, gemäß dem automatisch ausgewählten Steuerprogramm zur Steuerung der elektrischen Impulse, die aus der Energie von der Energieversorgungseinheit 18 und mittels Steuerung durch die Steuereinheit 15 bereitgestellt werden.

Insbesondere kann das automatisch ausgewählte Steuerprogramm eine Frequenz, eine Pulsweite, einer Modulation, eine Einschaltzeit und/oder eine Ausschaltzeit der elektrischen Impulse vorgeben, und zwar gemäß dem zu stimulierenden Körperteil 13b, welches der Nutzer ausgewählt hat. Dadurch kann der Nutzer auch ohne Vorwissen das System 10 intuitiv und schnell in Betrieb nehmen sowie gemäß einem auf das von ihm gewünschte Körperteil 13b abgestimmten Steuerprogramm betreiben.

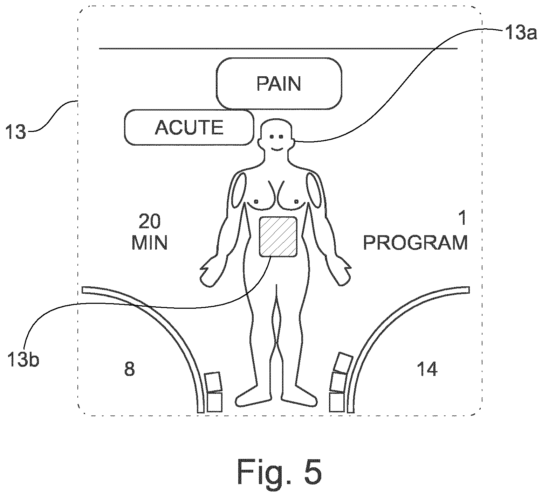

Figur 4 zeigt eine Draufsicht auf ein alternatives System 10 zur Stimulation von Körperregionen eines Nutzers. Im Unterschied zur Fig. 1 sind die Anzeige 13 und die Bedieneinheit 12 hier anders eingerichtet. So zeigt die Anzeige 13 einen anders gestalteten Anzeigeninhalt, der in der Figur 5 beispielhaft für eine ausgewählte Körperregion 13b im Bereich des Bauchs beim menschlichen Körper 13a illustriert ist, wo entsprechend die Elektrodeneinheiten 14a im Betrieb angeordnet sein können. Nach entsprechender Auswahl von TENS als Stimulationsprogramm und akut bzw. "ACUTE" als Unterstimulationsprogramm wird dem Nutzer auf der Anzeige 13 der Fig. 5 entsprechend angezeigt, dass eine Schmerzbehandlung durch TENS erfolgt, was durch Anzeige von "PAIN" für Schmerz angezeigt wird.

Ferner sind die Auswahl-Tastenfelder 12a, 12b, 12c, 12d beim Handgerät 11 der Fig. 4 anders eingerichtet. So sind die ersten Auswahl-Tastenfelder 12a, 12b nebeneinander angeordnet und unterhalb der nebeneinander angeordneten zweiten Auswahl-Tastenfelder 12c, 12d.

Ergänzend sei darauf hingewiesen, dass "umfassend" und "aufweisend" keine anderen Merkmale oder Schritte ausschließt und die unbestimmten Artikel "eine" oder "ein" keine Vielzahl ausschließen. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkungen anzusehen.

Ausdrücke wie "basierend auf", "in Beziehung stehend", "assoziiert" und ähnliche Ausdrücke nicht ausschließlich im Hinblick auf die Einheiten, Elemente und/oder Schritte zu verstehen, auf die sie sich beziehen, sofern nicht anders angegeben. Stattdessen sind diese Ausdrücke, sofern nicht anders angegeben, in dem Sinne zu verstehen, dass beispielsweise eine Einheit, ein Element oder ein Schritt, auf den sich einer dieser Ausdrücke oder ein ähnlicher Ausdruck bezieht, z. B. "basierend auf" einer oder einer anderen Einheit, einem Element oder einem Schritt, nicht ausschließt, dass die betreffende Einheit, das betreffende Element oder der betreffende Schritt auch auf einer anderen Einheit, einem anderen Element oder einem anderen Schritt als demjenigen, auf den er sich bezieht, "basieren" kann.

Jegliche Bezeichnung von Verfahren, Schritten und Elementen als erste, zweite usw., soweit hierin angegeben, dient lediglich dazu, die Verfahren, ihre Schritte und Elemente referenzierbar und voneinander unterscheidbar zu machen. Die Bezeichnung von Methoden, Schritten und Elementen stellt keineswegs eine Einschränkung des Umfangs dieser Offenbarung dar. Wenn in dieser Offenbarung beispielsweise ein dritter Schritt eines Verfahrens beschrieben wird, muss ein erster oder zweiter Schritt des Verfahrens nicht vorhanden sein und schon gar nicht vor dem dritten Schritt durchgeführt werden, es sei denn, es wird ausdrücklich darauf hingewiesen, dass sie per se oder vor dem dritten Schritt erforderlich sind. Darüber hinaus soll die Darstellung von Verfahren oder Schritten in einer bestimmten Reihenfolge lediglich ein Verständnis dieser Offenbarung erleichtern und stellt keineswegs eine Einschränkung des Umfangs dieser Offenbarung dar. Im Allgemeinen können die Verfahren und Schritte in jeder denkbaren Reihenfolge durchgeführt werden, es sei denn, es wird keine ausdrücklich vorgeschriebene Reihenfolge genannt. Insbesondere werden die Begriffe "erster", "zweiter", "dritter" oder "a)", "b)", "c)" und dergleichen in der Beschreibung und in den Ansprüchen zur Unterscheidung ähnlicher Elemente und nicht unbedingt zur Beschreibung einer sequentiellen oder chronologischen Reihenfolge verwendet. Es ist davon auszugehen, dass die so verwendeten Begriffe unter geeigneten Umständen austauschbar sind und dass die hierin beschriebenen Ausführungsformen der Offenbarung auch in anderen als den hierin beschriebenen oder abgebildeten Reihenfolgen funktionieren können.

## Patentansprüche

1. System (10) zur Stimulation, insbesondere zur wahlweise transkutanen elektrischen Nervenstimulation, TENS, elektrischen Muskelstimulation, EMS, und/oder Massagestimulation, von Körperregionen eines Nutzers, wobei das System (10) aufweist:
zumindest eine Elektrodeneinheit (14a), welche zur Anordnung an der Haut des Nutzers und zur Übertragung von elektrischen Impulsen an die Haut eingerichtet ist,
eine Bedieneinheit (12), welche zum Erfassen zumindest einer Bedieneingabe eines Nutzers zur Auswahl einer zu stimulierenden Körperregion (13b) aus einer Mehrzahl von zur Stimulation auswählbaren Körperregionen eingerichtet ist, und
eine Steuereinheit (15), welche zur Steuerung der elektrischen Impulse gemäß der zumindest einen Bedieneingabe derart eingerichtet ist, dass abhängig von der ausgewählten zu stimulierenden Körperregion (13b) automatisch ein Steuerprogramm zur Steuerung der elektrischen Impulse ausgewählt wird.

2. System (10) nach Anspruch 1, wobei die Bedieneinheit (12) ferner zum Erfassen zumindest einer Bedieneingabe eines Nutzers zur Auswahl einer Stimulationskategorie aus einer Mehrzahl von Stimulationskategorien eingerichtet ist, wobei das Steuerprogramm ferner abhängig von der ausgewählten Stimulationskategorie automatisch ausgewählt wird.

3. System (10) nach Anspruch 2, wobei die Mehrzahl von Stimulationskategorien zumindest zwei der folgenden aufweist: TENS, EMS und/oder Massagestimulation.

4. System (10) nach Anspruch 2 oder 3, wobei die Bedieneinheit (12) ferner zum Erfassen zumindest einer Bedieneingabe eines Nutzers zur Auswahl einer Unterstimulationskategorie aus zwei Unterstimulationskategorien eingerichtet ist, wobei das Steuerprogramm ferner abhängig von der ausgewählten Unterstimulationskategorie ausgewählt wird, wobei die zumindest zwei Unterstimulationskategorien eine chronische Unterstimulationskategorie und eine akute Unterstimulationskategorie umfassen.

5. System (10) nach einem der voranstehenden Ansprüche, wobei das automatisch ausgewählte Steuerprogramm eine Frequenz, eine Pulsweite, einer Modulation, eine Einschaltzeit und/oder eine Ausschaltzeit der elektrischen Impulse vorgibt.

6. System (10) nach einem der voranstehenden Ansprüche, wobei die zur Stimulation auswählbaren Körperregionen in der Steuereinheit (15) vorgegebene Körperregionen sind, welche zusammenhängende Körperpartien und/oder voneinander getrennte Körperpartien des Nutzers umfassen.

7. System (10) nach einem der voranstehenden Ansprüche, wobei die Bedieneinheit (12) von einem Endgerät des Nutzers, einem Handgerät (11) oder einem Tischgerät umfasst ist.

8. System (10) nach einem der voranstehenden Ansprüche, wobei die Bedieneinheit (12) mit der zumindest einen Elektrodeneinheit (14a) verbindbar oder verbunden ist und die Bedieneinheit (12) dazu eingerichtet ist, die elektrischen Impulse zu erzeugen und über die Verbindung mit der zumindest einen Elektrodeneinheit (14a) an die Haut zu übertragen.

9. System (10) nach einem der voranstehenden Ansprüche, wobei die Bedieneinheit (12) eine Anzeige (13) aufweist, auf welcher die zur Stimulation auswählbaren Körperregionen in Abhängigkeit von der zumindest einen Bedieneingabe des Nutzers an der Bedieneinheit (12) angezeigt werden.

10. System (10) nach einem der voranstehenden Ansprüche, wobei die Bedieneinheit (12) eine Mehrzahl von Tastenfeldern (12a, 12b, 12c, 12d, 12e, 12f) zur Erfassung der zumindest einen Bedieneingabe des Nutzers aufweist.

11. System (10) nach Anspruch 10, wobei die Bedieneinheit zwei erste Auswahl-Tastenfelder (12a, 12b) zur Auswahl der zu stimulierenden Körperregion (13b) und ein Bestätigungs-Tastenfeld (12e) zur Bestätigung der ausgewählten Körperregion (13b) und/oder zur Einstellung von einem Stimulationsparameter in dem automatisch ausgewählten Steuerprogramm aufweist.

12. System (10) nach Anspruch 11, wobei die Bedieneinheit zwei zweite Auswahl-Tastenfelder (12c, 12d) aufweist.

13. Verfahren (100) zur Steuerung eines Systems (10) nach einem der voranstehenden Ansprüche zur Stimulation einer Körperregion eines Nutzers, wobei das Verfahren aufweist:
- Erfassen, an der Bedieneinheit (12), zumindest einer Bedieneingabe eines Nutzers zur Auswahl einer zu stimulierenden Körperregion (13b) aus einer Mehrzahl von zur Stimulation auswählbaren Körperregionen,
- automatisches Auswählen, durch die Steuereinheit (15), eines Steuerprogramms zur Steuerung der elektrischen Impulse abhängig von der ausgewählten zu stimulierenden Körperregion (13b), und
- Steuern der Steuereinheit (15) gemäß dem automatisch ausgewählten Steuerprogramm.

14. Computerprogramm, umfassend Befehle, die bewirken, dass das System (10) das Verfahren (100) nach Anspruch 13 ausführt.

15. Computerlesbares Speichermedium (17), auf dem das Computerprogramm nach Anspruch 14 gespeichert ist.
